# EUROPEAN PATENT SPECIFICATION

(11) **EP 3 348 260 B1**
(45) Date of publication and mention of the grant of the patent: **02.06.2021**
(21) Application number: 16843709.3
(22) Date of filing: 05.09.2016
(51) Int. Cl.: A61K 31/137, A61K 9/00, A61K 9/08, A61K 47/38, A61P 27/02, A61K 47/36

(54) **PHARMACEUTICAL TRAMADOL COMPOSITION FOR OPHTHALMIC USE**
PHARMAZEUTISCHE TRAMADOLZUSAMMENSETZUNG ZUR OPHTHALMISCHEN VERWENDUNG
COMPOSITION PHARMACEUTIQUE DE TRAMADOL DESTINÉE À UN USAGE OPHTHALMIQUE

(30) Priority: 09.09.2015 ES 201500657
(43) Date of publication of application: 18.07.2018
(73) Proprietor: Farmalider, S.A., 28108 Alcobendas (Madrid) (ES)
(72) Inventor: SANZ MENENDEZ, Nuria, 28108 Alcobendas-Madrid (ES); HORCAJADA CORDOBA, Raquel, 28108 Alcobendas-Madrid (ES); MARTINEZ-ALZAMORA, Fernando, 28108 Alcobendas-Madrid (ES); HERRERO VANRELL, Rocío, 28108 Alcobendas-Madrid (ES); BENITEZ DEL CASTILLO, José Manuel, 28108 Alcobendas-Madrid (ES)
(74) Representative: ABG Intellectual Property Law, S.L.
(86) International application number: PCT/ES2016/000092
(87) International publication number: WO 2017/042404

(56) References cited:
- WO-A1-2013/150277
- WO-A2-2012/136969
- ES-A1- 2 540 151

## Description

### Field of the Invention

The present invention relates to pharmaceutical compositions for their local administration by ophthalmic route, that are intended for the treatment of ocular pain, and especially, to compositions containing tramadol as active ingredient.

### State of the prior art

Ocular pain may be described as a burning, throbbing, aching, or stabbing sensation in or around the eye, or as a feeling like there was a foreign body lodged in the eye.

Ocular pain can be originated, for instance, as a result of traumatic injuries or surgery of the eye area, or as a result of infections, inflammation, eye dryness, neuropathies, problems with contact lenses or eyestrain.

Among the possible causes of ocular pain, we can mention post-surgical states after ophthalmic surgery, for example after cataract surgery or refractive surgery.

The topical administration of drugs is the preferred route for the treatment of ocular pain, since it provides a greater concentration of the drug locally in the affected area, whilst avoiding the undesired systemic effects associated with oral administration.

Among the various drugs suitable for being used by ophthalmic route for the treatment of ocular pain we can basically highlight the non-steroidal anti-inflammatory drugs (NSAIDs) and local anaesthetics.

NSAIDs have been in widespread use in ophthalmology since several years ago, as described, for example, in the review article of Kim et al. Nonsteroidal Anti-inflammatory drugs in ophthalmology, Survey Ophthalmol., 2010. 55 (2), p108-133, highlighting the use of NSAIDs in topical form, e.g. to reduce myosis and inflammation in eye surgery, for the treatment of scleritis, prevention and treatment of macular oedema associated with cataract surgery, to alleviate postoperative pain and photophobia associated with refractive surgery and to reduce the itching associated with allergic conjunctivitis.

Thus, various eyedrops have been marketed based on NSAIDs as active ingredients, mainly with bromfenac, diclofenac, flurbiprofen, ketorolac and nepafenac.

Other topical drugs described in the state of the art for the treatment of ocular pain are local anaesthetics, like tetracaine, procaine, benoxinate or proparacaine.

However, the use of these products is not fully satisfactory, both as a result of their limited efficiency for the treatment of acute ocular pain and for the risk of certain undesired effects associated to their use.

In general, the most frequent adverse affect of eyedrops based on NSAIDs are generally mild, of the type of ocular burning, conjunctival hyperaemia or hypersensitivity reactions.

In some cases, however, when the patients are treated with high doses or during prolonged periods of time, the use of topical NSAIDs may cause more serious complications, mainly keratitis, corneal thinning, corneal erosions, corneal ulcerations and corneal perforations, as described, for example, in the article by Guídera et al. Keratitis, ulceration, and perforation associated with topical nonsteroidal anti-inflammatory drugs. Ophthalmology 2001, 108 (5), p936-44.

On the other hand, the repeated or extended use of local anaesthesia is associated with damaging effects for the corneal epithelium and on some occasions, they may cause more serious toxic effects, such as corneal infiltration, ulceration or even corneal perforation, as described in the article by McGee et al. Toxicities of topical ophthalmic anaesthetics. Review. Expert Opin. Drug Safety, 2007, 6 (6), p637-640.

Tramadol is a synthetic opioid which acts as a centrally acting pain reliever, according to a dual action mechanism: as an opioid receptor agonist and as a noradrenaline and serotonin reuptake inhibitor, so that both mechanisms of action, opiod and non-opioid, seem to operate synergistically. Also, tramadol is generally well-tolerated, and it is frequently used as the first choice drug for the treatment of post-surgery pain, instead of NSAIDs and other opioid drugs, like morphine, as described for example, in the article by Lehmann KA, Le tramadol dans les douleurs aiguës, Drugs, 1997, 53 (2), Supplement, p25-33. Tramadol is usually administered orally, rectally and parenterally.

On some occasions, tramadol has also been suggested for topical ophthalmic administration, as an alternative analgesic for the treatment of ophthalmic pain.

Thus, patent application WO-A-2012/136969 describes compositions intended for ophthalmic use, containing tramadol in a concentration of at least 0.5%, along with polymeric ophthalmic lubricating media, that must be unavoidably used, since the narcotic effect of tramadol leads to a reduction in blinking and the resulting eye dryness.

Thus, examples 6 to 9 of such patent application describe the administration of ophthalmic eye-drop formulations containing 0.5% or 1.0% of tramadol for the treatment of patients suffering from ophthalmic pain caused by infection, allergic diseases, dry eye and blepharitis, respectively, highlighting that the most effective results are achieved at a concentration of 1.0%.

Despite the different alternatives described so far, there is still the need to have a composition for topical ophthalmic use for the treatment of ocular pain, which is therapeutically effective and involves a lower risk of undesired secondary effects.

### Object of the invention

The object of the present invention is an ophthalmic pharmaceutical composition comprising tramadol.

Part of the object of the invention is also the use of said composition for the treatment of ocular pain.

### Detailed description of the invention

The object of the present invention is an aqueous ophthalmic pharmaceutical composition comprising tramadol or a pharmaceutically acceptable salt thereof, wherein the concentration of tramadol is comprised between 0.04% (w/v) and 0.07% (w/v), expressed as equivalent concentration of tramadol hydrochloride, and wherein tramadol is the only active ingredient of the composition.

The authors of the present invention have developed an aqueous ophthalmic pharmaceutical composition comprising tramadol, which is surprisingly very effective for the treatment of ocular pain, even though such active ingredient is found at very low concentrations, specifically between 0.04 and 0.07% (w/v), and consequently, such composition is also especially safe and substantially has no adverse effects.

In this description, and unless otherwise specified, concentrations expressed as percentages always refer to weight/volume (w/v) percentages, or in other words, the weight of a specific component in grams per 100 ml of composition.

### Tramadol

Tramadol is the International Nonproprietary Name (INN) by which the compound (±)-cis-2-[(dimethylamino) methyl]-1-(3-methoxyphenyl)-cyclohexanol is known.

Tramadol is a drug belonging to the group of the opioids, although its mechanism of action is usually classified as an "atypical" opioid since it is neither completely opioid nor completely non-opioid. Tramadol has a dual action mechanism since, on the one hand, it acts on the µ-opioid receptors to which it bonds with low affinity, and on the other, it inhibits the recapturing of noradrenaline and serotonine, so that it increases the concentration of these neurotransmitters in localized areas of the brain, decreasing the pain threshold, as described, for example, in the article by Raffa et al., Opioid and nonopioid components independently contribute to the mechanism of action of tramadol, an 'atypical' opioid analgesic, J. Pharmacol. Exp. Ther., 1992, 260 (1), p275-85.

Tramadol is commercially distributed and can be prepared, for example, according to the process described in the U.S. Pat. No. 3,652,589.

In the context of the present invention, the term tramadol includes, in a broad sense, any of its solvates, polymorphs, stereoisomers, mixtures of stereoisomers and racemic forms.

Pharmaceutically acceptable salts of tramadol relate to the addition of salts to acids, which can be prepared according to conventional methods well known by a person skilled in the art, using pharmaceutically acceptable and substantially non-toxic organic or inorganic acids. Said acids include hydrochloric acid, nitric acid, sulphuric acid, phosphoric acid, acetic acid, propionic acid, maleic acid, malonic acid, succinic acid, citric acid, tartaric acid, malic acid, salicylic acid or phthalic acid, among others. Preferably, hydrochloric acid is used.

In a preferred embodiment of the invention, the tramadol is in the form of its hydrochloride salt, or tramadol hydrochloride. More preferably, it is found in the form of tramadol hydrochloride, in racemic form.

The concentration of tramadol in the ophthalmic pharmaceutical composition of the invention is comprised between 0.04% and 0.07%, preferably comprised between 0.05% and 0.06%, expressed as equivalent concentration of tramadol hydrochloride.

The composition only contains tramadol as its active ingredient.

Throughout the present description, the concentrations of tramadol in the ophthalmic composition of the invention always relate to the equivalent concentration of tramadol hydrochloride, irrespective of whether the tramadol is used in the form of free base or in the form of another salt. Thus, for example, a concentration of tramadol of 0.05% expressed as equivalent concentration of tramadol hydrochloride approximately corresponds to a concentration of 0.044% of tramadol as free base, as any person skilled in the art already knows how to calculate, depending on the molecular weight of said substances.

### Ophthalmic composition

The pharmaceutical composition according to the present invention is an aqueous composition which is intended for topical ophthalmic administration, i.e., which is designed for its local application in the eye or adjacent areas, according to the definition provided by the European Pharmacopoeia where ophthalmic compositions are defined as sterile preparations (liquid, semisolid or solid) intended for their administration on the eyeball or the conjunctiva, or for their insertion in the conjunctival sac.

The general characteristics of said ophthalmic compositions are well known for a person skilled in the art. Thus, for example, in the chapter *"Ophthalmic Preparations",* which corresponds to chapter 43 of the recognised manual of pharmaceutical technology *"*Remington The Science and Practice of Pharmacy", 20th edition, Lippincott, Williams & Wilkins, Philadelphia, 2000 [ISBN: 0-683-306472], the characteristics, formulation, mode of use and form of preparation of said compositions are described in detail.

The ophthalmic composition according to the present invention is topically administered on the eye surface, for example, in the form of drops or by spraying. Alternatively, it can also be administered by subconjunctival injection or retrobulbar injection, as described in the aforementioned chapter *"Ophthalmic Preparations".*

Preferably, the composition of the invention is topically administered on the eye surface, including both direct administration on said surface and its deposit on the edges of the eyelids, as is already known by any person skilled in the art and as described in the aforementioned chapter. For example, the composition can be applied with the help of a dropper, being instilled in the lower conjunctival sac, which is accessed by gently pulling the lower eyelid downwards forming a sac where the drop is deposited.

The ophthalmic composition according to the present invention is an aqueous formulation, i.e., the vehicle is water.

The composition according to the invention may be in the form of a solution or a suspension. In the ophthalmic solution all the ingredients are completely dissolved in the aqueous medium, whilst in the ophthalmic suspension, it is a dispersion in the aqueous vehicle of finely divided particles of the drug, relatively insoluble.

Preferably, the ophthalmic composition according to the present invention is in the form of solution.

The composition may optionally comprise a viscosity enhancer, and may typically present a liquid appearance, or slightly gelled, depending on the degree of viscosity.

The ophthalmic composition of the invention may additionally contain other optional ingredients.

### Viscosity enhancer

The composition according to the invention may optionally contain a viscosity enhancer.

A viscosity enhancer is a substance which increases the viscosity of the composition, which allows to increase the time that the active ingredient is in contact with the eye surface, which in turn may favour its absorption. The viscosity enhancer may eventually have a muco-adhesive and/or mucomimetic effect which means that there is an interaction between such enhancer and the mucins of the cornea and/or the conjunctiva, which also extends the time that the composition remains at the eye surface and enhances the absorption of the active ingredient. Additionally, the viscosity enhancer may also have an eye lubricating effect.

In a preferred embodiment of the invention, the composition comprises a viscosity enhancer.

The viscosity of the composition according to the invention is generally comprised between 1 and 100 cP, preferably between 2 and 50 cP, more preferably comprised between 3 and 40 cP, even more preferably comprised between 4 and 30 cP, and even more preferably comprised between 5 and 20 cP. 1 cP = 1 mPa·s.

In terms of its greater or lower viscosity, the ophthalmic composition according to the invention may present a completely liquid or a slightly gelled appearance.

Viscosity enhancers that are most suitable for their incorporation into the ophthalmic composition comprise, for instance, cellulosic derivatives and their eventual associations, including methyl cellulose, hydroxymethyl cellulose, hydroxyethyl cellulose, hydroxypropyl cellulose, hydroxypropyl methyl cellulose, sodium carboxymethyl cellulose, among others, polyvinyl alcohol, carbomer, polycarbophil, polyacrylamide, polyoxyethylene, polyvinylpyrrolidone, hyaluronic acid, sodium hyaluronate, sodium salt of chondroitin sulfate, Gellan gum, Xanthan gum, carrageenan, sodium alginate, pectin, or their mixtures.

Any person skilled in the art will have no difficulty in formulating the ophthalmic composition, eventually using a viscosity enhancer in the appropriate proportions to provide the composition with an optimum degree of viscosity and/or mucoadherence.

In a preferred embodiment, the viscosity enhancer is chosen among those that present the best mucoadhesive properties, so that it is preferably chosen among methyl cellulose, hydroxyethyl cellulose, hydroxypropyl cellulose, hydroxypropyl methyl cellulose, sodium carboxymethyl cellulose, sodium alginate, carrageenan, guar gum, carbomer, polyacrylamide, polycarbophil, hyaluronic acid, sodium hyaluronate and their mixtures.

In a particularly preferred embodiment, the viscosity enhancer is chosen among the group comprising hydroxypropyl methyl cellulose, sodium carboxymethyl cellulose, hyaluronic acid, sodium hyaluronate and their mixtures.

The viscosity enhancer is generally added in a proportion comprised between 0.1% and 1.0%, preferably comprised between 0.2% and 0.8%, in the case of hydroxypropyl methyl cellulose, between 0.1% and 1,0% in the case of sodium carboxymethyl cellulose, and between 0.1% and 0.5% in the case of hyaluronic acid or sodium hyaluronate.

### Isotonizing agent

The composition optionally contains an agent to regulate the osmolality, or isotonizing agent, which is chosen, for example, among the group comprised by sodium chloride, potassium chloride, sodium sulphate, potassium nitrate, mannitol, glycerol, propylene glycol sorbitol, glucose, dextrose, sucrose, and their mixtures. Preferably, the isotonizing agent used is sodium chloride.

The role of the isotonizing agent is to modify the osmolality of the composition, adapting it to the tonicity characteristics of the eye, to avoid discomfort in its application, so that it is preferably isotonic with respect to the tear fluid of the eye, or slightly hypertonic or hypotonic.

The composition according to the present invention generally has an osmolality comprised between 200 and 330 mOsm/Kg. Preferably, the osmolality of the composition is comprised between 280 mOsm/Kg and 330 mOsm/Kg, more preferably comprised between 290 mOsm/Kg and 320 mOsm/Kg, even more preferably comprised between 295 mOsm/Kg and 305 mOsm/Kg, and even more preferably, of approximately 300 mOsm/Kg, for all the indications proposed, with the exception of the hyperosmolar dry eye syndrome, where the osmolality of the composition of the present invention is preferably comprised between 200 mOsm/Kg and 320 mOsm/Kg, more preferably comprised between 220 mOsm/Kg and 280 mOsm/Kg, even more preferably comprised between 240 mOsm/Kg and 260 mOsm/Kg, and even more preferably of approximately 250 mOsm/Kg.

In case that an isotonizing agent is added to the composition, this must be added in the appropriate proportion to obtain osmolality values comprised within the range of the specified preferred values.

In a preferred embodiment of the invention, the composition contains sodium chloride as isotonizing agent in a proportion comprised between 0.5% and 1.5%, more preferably comprised between 0.75% and 1.25%.

### pH regulating agent

The composition according to this invention optionally contains an agent to regulate the pH which is ophthalmically acceptable. This agent is used to adjust the pH value of the composition in line with the preferred values, normally in the vicinity of the physiologic pH of tear fluid.

The pH of the composition is generally comprised between 6.0 and 8,0, preferably between 6.7 and 7,8, more preferably between 7.0 and 7.7, even more preferably between 7.3 and 7.5, and even more preferably, the composition has a pH of 7.4.

Ophthalmically acceptable agents used to regulate the pH are well known for a person skilled in the art and include, without limitation, acids such as hydrochloric acid, acetic acid, citric acid or boric acid; and bases such as sodium hydroxide, sodium phosphate or sodium citrate. A buffering system can also be used which is acceptable for its ophthalmic application, to regulate the pH in the required values, that are also well known for a person skilled in the art, for example acetate buffer, citrate buffer, phosphate buffer, borate buffer or bicarbonate buffer or their mixtures.

A person skilled in the art will have no problem to choose the appropriate pH regulating agent and incorporate it in the required amount to obtain a pH value within the aforementioned preferred ranges.

In a preferred embodiment of the invention, the compound used to adjust the pH value will be sodium hydroxide and/or hydrochloric acid.

### Preservatives

Optionally, the composition of the invention contains an opthalmologically acceptable preservative ingredient, especially when the composition is disposed in typical multi-dose containers, to avoid the microbial contamination of the composition once the container has been opened.

In general, the presence of a preservative is not necessary when single-dose containers or multi-dose containers which maintain their content sterile, or containers including a 0.22 micron filter for the dispensing of a sterile product are used.

Among the preservatives that may be most suitably used in the ophthalmic composition according to the present invention it is worth mentioning, for example, the compounds of quaternary ammonium such as benzalkonium chloride, benzethonium chloride, cetylpyridinium chloride, cetrimonium chloride, cetrimonium bromide or the quaternary amine polymers known as Polyquaternium-1 (commercially available under the name Polyquad®, Alcon); the organomercuric compounds, such as phenylmercuric nitrate, phenylmercuric acetate, phenylmercuric borate or thimerosal; parabens, such as methylparaben or propylparaben; alcohols or substituted phenols, such as chlorobutanol, phenethyl alcohol, or benzyl alcohol; or others such as chlorhexidine, sorbic acid, potassium sorbate, sodium benzoate, sodium propionate or sodium perborate; as well as certain commercially-available preservatives, including the so-called SofZia®, which contains borate, sorbitol, propylene glycol and zinc; or their mixtures.

Preferably, the preservative is chosen among the quaternary amine polymers known as Polyquaternium-1 (commercially available under the name Polyquad®, Alcon); and some commercially available preservative compositions, including the one known as SofZia®, which contains borate, sorbitol, propylene glycol and zinc.

### Other components

Optionally, the composition of the invention comprises a chelating agent, for example, sodium citrate or edetate salts, such as disodium edetate, disodium calcium edetate, trisodium edetate, or dipotassium edetate, or their mixtures. Optionally, the composition of the invention comprises an antioxidant. Among the antioxidants suitable for being used in the composition according to the present invention are, for example, sodium bisulphite, ascorbic acid or acethylcisteine, or their mixtures.

Likewise, the composition of the invention optionally comprises a surfactant whose typical function is favouring the contact of the active ingredient with the eye surface, as it helps to decrease the surface tension of the liquid and favours its penetration

Among the surfactants suitable for being used in the composition of the invention are, for example, fatty esters of sorbitan polyoxyethylene (polysorbates), alkylphenol ethoxylates, ethylene glycol-propylene glycol block copolymers, lecithins or phospholipids.

Reference is made here to the recognised manual of excipients for pharmaceutical use, the *"*Handbook of Pharmaceutical Excipients" by Rowe RC, Sheskey P J and Quinn M E, sixth edition, 2009, where the definitions and characteristics of the different ingredients mentioned herein can be found.

In an embodiment of the invention, the aqueous ophthalmic pharmaceutical composition comprises:
- tramadol or a pharmaceutically acceptable salt thereof, preferably tramadol hydrochloride, wherein the concentration of tramadol is comprised between 0.04% and 0.07%, preferably comprised between 0.05% and 0.06%, expressed as equivalent concentration of tramadol hydrochloride;
- an isotonizing agent, chosen among the group comprised by sodium chloride, potassium chloride, sodium sulphate, potassium nitrate, mannitol, glycerol, propylene glycol sorbitol, glucose, dextrose, sucrose, and their mixtures. Preferably, the isotonizing agent used is sodium chloride.
- A viscosity enhancer chosen among metyl cellulose, hydroxymethyl cellulose, hydroxyethyl cellulose, hydroxypropyl cellulose, hydroxypropyl methyl cellulose, sodium carboxymethyl cellulose, polyvinyl alcohol, carbomer, polycarbophil, polyacrylamide, polyoxyethylene, polyvinylpyrrolidone, hyaluronic acid, sodium hyaluronate, sodium salt of chondroitin sulfate, Gellan gum, Xanthan gum, carrageenan, sodium alginate, pectin, or their mixtures, and preferably chosen among methyl cellulose, hydroxyethyl cellulose, hydroxypropyl cellulose, hydroxypropyl methyl cellulose, sodium carboxymethyl cellulose, sodium alginate, carrageenan, guar gum, carbomer, polyacrylamide, polycarbophil, hyaluronic acid, sodium hyaluronate and their mixtures; and.
- Optionally, a pH regulating agent chosen among the group comprised by hydrochloric acid, acetic acid, citric acid or boric acid, sodium hydroxide, sodium phosphate or sodium citrate, acetate buffer, citrate buffer, phosphate buffer, borate buffer or bicarbonate buffer and their mixtures, and preferably among the group comprised of hydrochloric acid, sodium hydroxide and their mixtures;
   where the osmolality of the composition is comprised between 200 mOsm/Kg y 330 mOsm/Kg;
- Preferably comprised between 280 mOsm/Kg and 330 mOsm/Kg, more preferably comprised between 290 mOsm/Kg and 320 müsm/Kg, even more preferably comprised between 295 mOsm/Kg and 305 mOsm/Kg, and even more preferably, an osmolality of approximately 300 mOsm/Kg;
   or
- preferably comprised between 200 mOsm/Kg and 320 müsm/Kg, more preferably comprised between 220 mOsm/Kg and 280 mOsm/Kg, even more preferably comprised between 240 mOsm/Kg and 260 mOsm/Kg, and even more preferably, an osmolality of approximately 250 mOsm/Kg;
where the viscosity of the composition is generally comprised between 1 and 100 cP, preferably between 2 and 50 cP, more preferably comprised between 3 and 40 cP, even more preferably comprised between 4 and 30 cP, and even more preferably comprised between 5 and 20 cP, wherein 1 cP = 1mPa·s; where the pH of the composition is comprised between 6.0 and 8,0, preferably between 6.7 and 7,8, more preferably between 7.0 and 7.7, even more preferably between 7.3 and 7.5, and even more preferably, the composition has a pH of 7.4; and
where the composition contains tramadol as its only active ingredient.

In a particularly preferred embodiment of the invention, the aqueous ophthalmic pharmaceutical composition comprises:
- tramadol or a pharmaceutically acceptable salt thereof, preferably tramadol hydrochloride wherein the concentration of tramadol is comprised between 0.04% and 0.07%, preferably comprised between 0.05% and 0.06%, expressed as equivalent concentration of tramadol hydrochloride;
- sodium chloride, where the concentration of sodium chloride is comprised between 0.5% and 1,5%, and preferably comprised between 0.75% and 1.25%;
- hydroxypropyl methyl cellulose, in a concentration comprised between 0.2% and 0.8%, and preferably comprised between 0.3% and 0.5%
- optionally, a pH regulating agent chosen among hydrochloric acid, sodium hydroxide and their mixtures;
where the pH of the composition is comprised between 6.0 and 8,0, preferably between 6.7 and 7,8, more preferably between 7.0 and 7.7, even more preferably between 7.3 and 7.5, and even more preferably, the composition has a pH of 7.4; and
where the composition contains tramadol as its only active ingredient.

In another particularly preferred embodiment of the invention, the aqueous ophthalmic pharmaceutical composition comprises:
- tramadol or a pharmaceutically acceptable salt thereof, preferably tramadol hydrochloride wherein the concentration of tramadol is comprised between 0.04% and 0.07%, preferably comprised between 0.05% and 0.06%, expressed as equivalent concentration of tramadol hydrochloride;
- sodium chloride, where the concentration of sodium chloride is comprised between 0.5% and 1,5%, and preferably comprised between 0.75% and 1.25%;
- sodium carboxymethyl cellulose, in a concentration comprised between 0.1% and 1.0%, and preferably comprised between 0.5% and 0.8%
- optionally, a pH regulating agent chosen among hydrochloric acid, sodium hydroxide and their mixtures;
where the pH of the composition is comprised between 6.0 and 8,0, preferably between 6.7 and 7,8, more preferably between 7.0 and 7.7, even more preferably between 7.3 and 7.5, and even more preferably, the composition has a pH of 7.4; and
where the composition contains tramadol as its only active ingredient.

In another particularly preferred embodiment of the invention, the aqueous ophthalmic pharmaceutical composition comprises:
- tramadol or a pharmaceutically acceptable salt thereof, preferably tramadol hydrochloride wherein the concentration of tramadol is comprised between 0.04% and 0.07%, preferably comprised between 0.05% and 0.06%, expressed as equivalent concentration of tramadol hydrochloride;
- sodium chloride, where the concentration of sodium chloride is comprised between 0.5% and 1,5%, and preferably comprised between 0.75% and 1.25%;
- hyaluronic acid or sodium hyaluronate, in a concentration comprised between 0.1% and 0.5%, and preferably comprised between 0.2% and 0.4%
- optionally, a pH regulating agent chosen among hydrochloric acid, sodium hydroxide and their mixtures;
where the pH of the composition is comprised between 6.0 and 8,0, preferably between 6.7 and 7,8, more preferably between 7.0 and 7.7, even more preferably between 7.3 and 7.5, and even more preferably, the composition has a pH of 7.4; and
where the composition contains tramadol as its only active ingredient.

### Use of the ophthalmic composition containing tramadol

The efficiency of the ophthalmic composition according to the invention for the treatment of ocular pain was tested during a trial intended to determine the activity of the composition on the nerve endings of multimode sensory receptors and cold receptors of the cornea, as described in the article by Acosta et al., Comparative effects of the nonsteroidal anti-inflammatory drug nepafenac on corneal sensory nerve fibers responding to chemical irritation, Invest Ophthalmol Vis Sci. 2007 Jan; 48(1):182-8.

The authors of the invention have verified that the ophthalmic compositions according to the invention, which contain a low concentration of the active ingredient tramadol, are surprisingly very effective when used according to such model.

Thus, the object of the present invention also comprises the composition according to the invention for use in the topical treatment of ocular pain.

Alternatively, such object may be formulated as the composition according to the invention, to be used in the treatment of ocular pain through topical ophthalmic administration.

Within the scope of the present invention, the term "treatment" includes the treatment with therapeutic purpose, i.e. aimed at the elimination, reduction, improvement or relief of symptoms when they have already manifested themselves, and also includes the treatment with a preventive or prophylactic purpose, i.e. aimed at preventing or delaying the appearance of the ocular pain, or at reducing its incidence.

The treatment according to the use of the present invention is indicated for its application to any mammal animal which requires said treatment, and preferably humans.

Furthermore, the ocular pain according to the object of the present invention is understood in a broad sense, comprising both eye disorders, including all the parts of the eye, among them iris, crystalline lens, cornea, conjunctiva, sclera, choroid, retina, optic disc or vitreous humour, as well as in the surrounding areas, for example, the eyelids.

On the other hand, the aetiology of said pain can be diverse, for example, as a response to traumatisms or eye surgery, or due to infectious processes, allergies, immunological reactions, or due to other causes, all of them being included within the scope of the present invention.

Thus, the use which forms part of the present invention relates to the treatment of ocular pain associated to various diseases or pathological states, including, without limitation, the following:
- post-surgical states after eye surgery
- inflammatory states of diverse origin and localization, such as allergic conjunctivitis, viral conjunctivitis, bacterial conjunctivitis, blepharitis, uveitis, iridocyclitis, intermediate uveitis, chorioretinitis, scleritis, episcleritis, retinitis, or keratitis, among others;
- dry eye syndrome;
- eye injury due to traumatism, burn, radiation, introduction of a foreign body, or contact with chemicals; or
- use of contact lenses.

Within the framework of the present invention, the eye surgery includes any surgical technique performed in the eye, including, for example, cataract surgery, corneal transplant, occuloplasty, or any other refractive surgery technique. On the other hand, refractive surgery includes techniques such as radial keratotomy (RK), astigmatic keratotomy (AK), photorefractive keratectomy (PRK), or Laser-assisted in situ Keratomileusis (LASIK), which are all included within the field of the present invention. Among the causes of pain arisen as a consequence of eye surgery, macular edema after cataract surgery and photophobia after eye surgery, especially photophobia after refractive surgery should also be particularly included.

In a particularly preferred embodiment, the use according to the present invention relates to the treatment of ocular pain associated to post-surgical states after eye surgery.

In an even more preferred embodiment, the use according to the present invention relates to the treatment of ocular pain associated to post-surgical states after cataract surgery, radial keratotomy (RK), photorefractive keratectomy (PRK), or Laser-assisted In-Situ Keratomileusis (LASIK), including macular edema after cataract surgery and photophobia after radial keratotomy (RK), as well as photophobia after photorefractive keratectomy (PRK).

In another preferred embodiment, the use according to the present invention relates to the treatment of ocular pain associated to an eye injury.

In another preferred embodiment, the use according to the present invention relates to the treatment of ocular pain associated to dry eye syndrome.

### Preparation of the composition

The aqueous composition for ophthalmic use according to the present invention is prepared using standard procedures, which are well known for any person skilled in the art, as described, for example, in the chapter "Ophthalmic Formulations" of the aforementioned manual "Remington The Science and Practice of Pharmacy*".*

Thus, one of the requirements that the ophthalmic compositions must comply with is that they must be sterile. Different techniques can be used for the sterilization of the ophthalmic compositions of the invention, all of them sufficiently known by those skilled in the art, for example, with high-pressure steam at 121 °C. (in an autoclave), by sterilizing filtration, sterilization with ethylene oxide, or by radiation.

Preferably, the ophthalmic composition of the invention is sterilized by sterilizing filtration and/or with high-pressure steam at 121 °C.

The aqueous composition according to the invention can be prepared with purified water, according to the characteristics specified in the Spanish Royal Pharmacopoeia, second edition, or with water for injection, that are easily commercially available.

Purified water is usually obtained through distillation, ion exchange or any other appropriate means, using drinking water as a basis.

Water for injection is usually obtained through sterilization of distilled, pyrogen-free water.

Thus, the composition for ophthalmic use according to the present invention can be prepared, for example, according to a process comprising the dissolution or dispersion of tramadol or a salt thereof in purified water/water for injection, together with other possible optional ingredients, such as an isotonizing agent, a preservative agent, or a viscosity enhancer, among others, as described above, or a combination thereof.

The pH of the composition can be adjusted to a value comprised between 6.0 and 8.0, adding an appropriate pH regulating agent, if required.

Finally, if necessary, additional purified water/water for injection is added until the required final volume, and it can be also verified that the osmolarity values are comprised between the recommended values, i.e. between 200 and 330 mOsm/Kg and more preferably between 280 mOsm/Kg and 330 mOsm/Kg for all the suggested indication, with the exception of the hyperosmolar dry eye syndrome, where osmolality values should preferably range between 200 mOsm/Kg and 320 mOsm/Kg.

The solution obtained can be sterilized, for example, by sterilizing filtration.

Finally, the resulting solution can be dosed in multidose or single-dose containers, suitable for ophthalmic administration, and that are well known by any person skilled in the art of pharmaceutical technology. Optionally, said packages containing the composition of the invention are sterilized in an autoclave, for example, by treatment at 121 °C. for approximately 20 minutes.

The composition of the present invention can be also prepared under no sterile conditions, packing it into a container comprising a 0.22 micron filter, to obtain a sterile product at the time of administration.

A series of exemplary embodiments of the invention are shown below, for illustration purposes, but without limitation.

### Examples

### Example 1: Eyedrops containing 0.05% of tramadol hydrochloride

An aqueous solution was prepared using the ingredients detailed in the following table:

| **Ingredient** | **Quantity % (w/v)** |
|---|---|
| Tramadol HCl | 0.05 |
| NaCl | 0.90 |
| NaOH/HCl | q.s. pH 7.4 |
| Purified water | q.s. 100 ml |

Part of the purified water, in a quantity of approximately 75% of the total, was placed in a reactor. Then, under constant stirring, tramadol hydrochloride and sodium chloride were consecutively added until total dissolution.

The pH was then adjusted with NaOH/HCl to a value of 7.4, and the remainder of the purified water was added flushing until the final volume. The solution thus obtained was sterilized by filtration through a 0.22 micron filter.

The solution had an osmolality of 300 mOsm.

### Example 2: Eyedrops containing 0.05% of tramadol hydrochloride

An aqueous solution was prepared using the ingredients detailed in the following table:

| **Ingredient** | **Quantity % (w/v)** |
|---|---|
| Tramadol HCl | 0.05 |
| NaCl | 0.990 |
| Hydroxymethyl cellulose | 0.30 |
| NaOH/HCl | q.s. pH 7.4 |
| Purified water | q.s. 100 ml |

Part of the purified water, in a quantity of approximately 75% of the total, was placed in a reactor. Then, under constant stirring, tramadol hydrochloride, sodium chloride and an appropriate amount of a solution containing hydroxymethyl cellulose, previously dissolved in cold purified water were consecutively added until total dissolution.

The pH was then adjusted with NaOH/HCl to a value of 7.4, and the remainder of the purified water was added flushing until the final volume. The solution thus obtained was sterilized by filtration through a 0.22 micron filter.

The solution had an osmolality of 300 mOsm.

## Claims

1. An aqueous ophthalmic pharmaceutical composition comprising tramadol or a pharmaceutically acceptable salt thereof, **characterized in that** the concentration of tramadol is comprised between 0.04% (w/v) and 0.07% (w/v), expressed as equivalent concentration of tramadol hydrochloride, and wherein tramadol is the only active ingredient of the composition.

2. A composition according to claim 1, **characterized in that** tramadol is present in the form of its hydrochloride salt.

3. A composition according to claims 1 or 2, **characterized in that** it contains a viscosity enhancer chosen among the group comprised by methyl cellulose, hydroxymethyl cellulose, hydroxyethyl cellulose, hydroxypropyl cellulose, hydroxypropyl methyl cellulose, sodium carboxymethyl cellulose, polyvinyl alcohol, carbomer, polycarbophil, polyacrylamide, polyoxyethylene, polyvinylpyrrolidone, hyaluronic acid, sodium hyaluronate, sodium salt of chondroitin sulfate, Gellan gum, Xanthan gum, carrageenan, sodium alginate, pectin, and their mixtures.

4. A composition according to claim 3, **characterized in that** the viscosity enhancer is chosen among the group comprised by methyl cellulose, hydroxyethyl cellulose, hydroxypropyl cellulose, hydroxypropyl methyl cellulose, sodium carboxymethyl cellulose, sodium alginate, carrageenan, guar gum, carbomer, polyacrylamide, polycarbophil, hyaluronic acid, sodium hyaluronate and their mixtures.

5. A composition according to any of claims 1 to 4, **characterized in that** it contains an isotonizing agent chosen among the group comprised by sodium chloride, potassium chloride, sodium sulphate, potassium nitrate, mannitol, glycerol, propylene glycol sorbitol, glucose, dextrose, sucrose, and their mixtures.

6. A composition according to claim 5, **characterized in that** the isotonizing agent is sodium chloride.

7. A composition according to any of claims 1 to 6, **characterized in that** the pH of the composition is comprised between 6.0 and 8.0.

8. A composition according to any of claims 1 to 7, **characterized in that** it contains a preservative.

9. A composition according to claims 1 or 2, **characterized in that** it contains:
- tramadol or a pharmaceutically acceptable salt thereof, at a concentration comprised between 0.04% (w/v) and 0.07% (w/v), expressed as equivalent concentration of tramadol hydrochloride;
- sodium chloride, at a concentration comprised between 0.5% (w/v) and 1.5% (w/v); and
- hydroxypropyl methyl cellulose, at a concentration comprised between 0.2% (w/v) and 0.8% (w/v);

10. A composition according to claims 1 or 2, **characterized in that** it contains:
- tramadol or a pharmaceutically acceptable salt thereof, at a concentration comprised between 0.04% (w/v) and 0.07% (w/v), expressed as equivalent concentration of tramadol hydrochloride;
- sodium chloride, at a concentration comprised between 0.5% (w/v) and 1.5% (w/v); and
- sodium carboxymethyl cellulose, at a concentration comprised between 0.1% (w/v) and 1.0% (w/v);

11. A composition according to claims 1 or 2, **characterized in that** it contains:
- tramadol or a pharmaceutically acceptable salt thereof, at a concentration comprised between 0.04% (w/v) and 0.07% (w/v), expressed as equivalent concentration of tramadol hydrochloride;
- sodium chloride, at a concentration comprised between 0.5% (w/v) and 1.5% (w/v); and
- hyaluronic acid or sodium hyaluronate, at a concentration comprised between 0.1% (w/v) and 0.5% (w/v);

12. The composition according to any of the preceding claims 1 to 11 for use in the topical treatment of ocular pain.

13. The composition for use according to claim 12, **characterized in that** ocular pain is associated to:
- post-surgical states after eye surgery; or
- allergic conjunctivitis, viral conjunctivitis, bacterial conjunctivitis, blepharitis, uveitis, iridocyclitis, intermediate uveitis, chorioretinitis, scleritis, episcleritis, retinitis, or keratitis, or
- dry eye syndrome; or
- eye injury due to traumatism, burn, radiation, introduction of a foreign body, or
- contact with chemicals; or
- use of contact lenses.

14. The composition for use according to claim 13, **characterized in that** ocular pain is associated to post-surgical states after eye surgery, dry eye syndrome or ocular injury.

## Patentansprüche

1. Wässrige ophthalmische pharmazeutische Zusammensetzung, umfassend Tramadol oder ein pharmazeutisch annehmbares Salz davon, **dadurch gekennzeichnet, dass** die Konzentration an Tramadol im Bereich von 0,04% (w/v) bis 0,07% (w/v) liegt, ausgedrückt als Äquivalentkonzentration von Tramadol-Hydrochlorid, wobei Tramadol der einzige Wirkstoff der Zusammensetzung ist.

2. Zusammensetzung gemäß Anspruch 1, **dadurch gekennzeichnet, dass** Tramadol in Form seines Hydrochlorid-Salzes vorliegt.

3. Zusammensetzung gemäß Anspruch 1 oder 2, **dadurch gekennzeichnet, dass** sie ein Verdickungsmittel enthält, das aus der Gruppe ausgewählt ist, die aus Methylcellulose, Hydroxymethylcellulose, Hydroxyethylcellulose, Hydroxypropylcellulose, Hydroxypropylmethylcellulose, Natriumcarboxymethylcellulose, Polyvinylalkohol, Carbomer, Polycarbophil, Polyacrylamid, Polyoxyethylen, Polyvinylpyrrolidon, Hyaluronsäure, Natriumhyaluronat, dem Natriumsalz von Chondroitinsulfat, Gellan, Xanthan, Carrageen, Natriumalginat, Pektin und ihren Gemischen besteht.

4. Zusammensetzung gemäß Anspruch 3, **dadurch gekennzeichnet, dass** das Verdickungsmittel aus der Gruppe ausgewählt ist, die aus Methylcellulose, Hydroxyethylcellulose, Hydroxypropylcellulose, Hydroxypropylmethylcellulose, Natriumcarboxymethylcellulose, Natriumalginat, Carrageen, Guarkernmehl, Carbomer, Polyacrylamid, Polycarbophil, Hyaluronsäure, Natriumhyaluronat und ihren Gemischen besteht.

5. Zusammensetzung gemäß einem der Ansprüche 1 bis 4, **dadurch gekennzeichnet, dass** sie ein Isotonisierungsmittel enthält, das aus der Gruppe ausgewählt ist, die aus Natriumchlorid, Kaliumchlorid, Natriumsulfat, Kaliumnitrat, Mannit, Glycerin, Propylenglycolsorbit, Glucose, Dextrose, Saccharose und ihren Gemischen besteht.

6. Zusammensetzung gemäß Anspruch 5, **dadurch gekennzeichnet, dass** es sich bei dem Isotonisierungsmittel um Natriumchlorid handelt.

7. Zusammensetzung gemäß einem der Ansprüche 1 bis 6, **dadurch gekennzeichnet, dass** der pH-Wert der Zusammensetzung im Bereich von 6,0 bis 8,0 liegt.

8. Zusammensetzung gemäß einem der Ansprüche 1 bis 7, **dadurch gekennzeichnet, dass** sie einen Konservierungsstoff enthält.

9. Zusammensetzung gemäß Anspruch 1 oder 2, **dadurch gekennzeichnet, dass** sie enthält:
- Tramadol oder ein pharmazeutisch annehmbares Salz davon in einer Konzentration, die im Bereich von 0,04% (w/v) bis 0,07% (w/v) liegt, ausgedrückt als Äquivalentkonzentration von Tramadol-Hydrochlorid;
- Natriumchlorid in einer Konzentration, die im Bereich von 0,5% (w/v) bis 1,5% (w/v) liegt; und
- Hydroxypropylmethylcellulose in einer Konzentration, die im Bereich von 0,2% (w/v) bis 0,8% (w/v) liegt.

10. Zusammensetzung gemäß Anspruch 1 oder 2, **dadurch gekennzeichnet, dass** sie enthält:
- Tramadol oder ein pharmazeutisch annehmbares Salz davon in einer Konzentration, die im Bereich von 0,04% (w/v) bis 0,07% (w/v) liegt, ausgedrückt als Äquivalentkonzentration von Tramadol-Hydrochlorid;
- Natriumchlorid in einer Konzentration, die im Bereich von 0,5% (w/v) bis 1,5% (w/v) liegt; und
- Natriumcarboxymethylcellulose in einer Konzentration, die im Bereich von 0,1% (w/v) bis 1,0% (w/v) liegt.

11. Zusammensetzung gemäß Anspruch 1 oder 2, **dadurch gekennzeichnet, dass** sie enthält:
- Tramadol oder ein pharmazeutisch annehmbares Salz davon in einer Konzentration, die im Bereich von 0,04% (w/v) bis 0,07% (w/v) liegt, ausgedrückt als Äquivalentkonzentration von Tramadol-Hydrochlorid;
- Natriumchlorid in einer Konzentration, die im Bereich von 0,5% (w/v) bis 1,5% (w/v) liegt; und
- Hyaluronsäure oder Natriumhyaluronat in einer Konzentration, die im Bereich von 0,1% (w/v) bis 0,5% (w/v) liegt.

12. Zusammensetzung gemäß einem der vorstehenden Ansprüche 1 bis 11 zur Verwendung bei der topischen Behandlung von Augenschmerzen.

13. Zusammensetzung zur Verwendung gemäß Anspruch 12, **dadurch gekennzeichnet, dass** die Augenschmerzen assoziiert sind mit:
- postoperativen Zuständen nach einer Augenoperation; oder
- allergischer Konjunktivitis, viraler Konjunktivitis, bakterieller Konjunktivitis, Blepharitis, Uveitis, Iridocyclitis, intermediäre Uveitis, Chorioretinitis, Skleritis, Episkleritis, Retinitis oder Keratitis; oder
- Keratoconjunctivitis sicca; oder
- einer Augenverletzung aufgrund eines Traumas, einer Verbrennung, Verstrahlung, Einführung eines Fremdkörpers; oder
- einem Kontakt mit Chemikalien; oder
- der Verwendung von Kontaktlinsen.

14. Zusammensetzung zur Verwendung gemäß Anspruch 13, **dadurch gekennzeichnet, dass** die Augenschmerzen mit postoperativen Zuständen nach einer Augenoperation, Keratoconjunctivitis sicca oder einer Augenverletzung assoziiert sind.

## Revendications

1. Une composition pharmaceutique ophtalmique aqueuse comprenant du tramadol ou un des sels pharmaceutiquement acceptables de celui-ci, **caractérisée en ce que** la concentration en tramadol est comprise entre 0,04% (p/v) et 0,07% (p/v), exprimée en concentration équivalente de chlorhydrate de tramadol, et dans laquelle le tramadol est le seul ingrédient actif de la composition.

2. Une composition selon la revendication 1, **caractérisée en ce que** le tramadol est présent sous la forme de son sel chlorhydrate.

3. Une composition selon les revendications 1 ou 2, **caractérisée en ce qu'**elle contient un agent améliorant la viscosité choisi dans le groupe comprenant la méthylcellulose, l'hydroxyméthylcellulose, l'hydroxyéthylcellulose, l'hydroxypropylcellulose, l'hydroxypropylméthyl-cellulose, la carboxyméthylcellulose sodique, l'alcool polyvinylique, le carbomère, le polycarbophile, le polyacrylamide, le polyoxyéthylène, le polyvinylpyrrolidone, l'acide hyaluronique, l'hyaluronate de sodium, le sel de sodium de chondroïtine sulfate, la gomme gellane, la gomme xanthane, la carraghénane, l'alginate de sodium, la pectine et les mélanges de ceux-ci.

4. Une composition selon la revendication 3, **caractérisée en ce que** l'agent améliorant la viscosité est choisi parmi le groupe comprenant la méthylcellulose, l'hydroxyéthylcellulose, l'hydroxypropylcellulose, l'hydroxypropylméthylcellulose, la carboxyméthylcellulose sodique, l'alginate de sodium, la carraghénane, la gomme de guar, le carbomère, le polyacrylamide., le polycarbophile, l'acide hyaluronique, l'hyaluronate de sodium et les mélanges de ceux-ci.

5. Une composition selon l'une quelconque des revendications 1 à 4, **caractérisée en ce qu'**elle contient un agent isotonisant choisi dans le groupe constitué par le chlorure de sodium, le chlorure de potassium, le sulfate de sodium, le nitrate de potassium, le mannitol, le glycérol, le propylène glycol sorbitol, le glucose, le dextrose, la saccharose et les mélanges de ceux-ci.

6. Une composition selon la revendication 5, **caractérisée en ce que** l'agent isotonisant est le chlorure de sodium.

7. Une composition selon l'une quelconque des revendications 1 à 6, **caractérisée en ce que** le pH de la composition est compris entre 6,0 et 8,0.

8. Une composition selon l'une quelconque des revendications 1 à 7, **caractérisée en ce qu'**elle contient un conservateur.

9. Une composition selon les revendications 1 ou 2, **caractérisée en ce qu'**elle contient :
- du tramadol ou un sel pharmaceutiquement acceptable de celui-ci, à une concentration comprise entre 0,04% (p/v) et 0,07% (p/v), exprimée en concentration équivalente de chlorhydrate de tramadol ;
- du chlorure de sodium, à une concentration comprise entre 0,5% (p/v) et 1,5% (p/v) ; et
- de l'hydroxypropylméthylcellulose, à une concentration comprise entre 0,2% (p/v) et 0,8% (p/v).

10. Une composition selon les revendications 1 ou 2, **caractérisée en ce qu'**elle contient :
- du tramadol ou un sel pharmaceutiquement acceptable de celui-ci, à une concentration comprise entre 0,04% (p/v) et 0,07% (p/v), exprimée en concentration équivalente de chlorhydrate de tramadol ;
- du chlorure de sodium, à une concentration comprise entre 0,5% (p/v) et 1,5% (p/v) ; et
- du carboxyméthylcellulose sodique, à une concentration comprise entre 0,1% (p/v) et 1,0% (p/v).

11. Une composition selon les revendications 1 ou 2, **caractérisée en ce qu'**elle contient :
- du tramadol ou un sel pharmaceutiquement acceptable de celui-ci, à une concentration comprise entre 0,04% (p/v) et 0,07% (p/v), exprimée en concentration équivalente de chlorhydrate de tramadol ;
- du chlorure de sodium, à une concentration comprise entre 0,5% (p/v) et 1,5% (p/v) ; et
- de l'acide hyaluronique ou de l'hyaluronate de sodium, à une concentration comprise entre 0,1% (p/v) et 0,5% (p/v).

12. La composition selon l'une quelconque des revendications précédentes 1 à 11 pour une utilisation dans le traitement topique des douleurs oculaires.

13. La composition à utiliser selon la revendication 12, **caractérisée en ce que** la douleur oculaire est associée à :
- des états post-chirurgicaux après chirurgie oculaire ; ou
- une conjonctivite allergique, une conjonctivite virale, une conjonctivite bactérienne, une blépharite, une uvéite, une iridocyclite, une uvéite intermédiaire, une choriorétinite, une sclérite, une épisclérite, une rétinite ou une kératite, ou
- un syndrome de l'œil sec ; ou
- une lésion oculaire due à un traumatisme, une brûlure, un rayonnement, une introduction d'un corps étranger ou
- un contact avec des produits chimiques ; ou
- l'utilisation de lentilles de contact.

14. La composition pour une utilisation selon la revendication 13, **caractérisée en ce que** la douleur oculaire est associée à des états post-chirurgicaux après une chirurgie oculaire, à un syndrome de l'œil sec ou à une lésion oculaire.
